# EUROPEAN PATENT SPECIFICATION

(11) **EP 3 426 279 B1**
(45) Date of publication and mention of the grant of the patent: **04.05.2022**
(21) Application number: 17711776.9
(22) Date of filing: 06.03.2017
(51) Int. Cl.: A61K 31/137, A61K 31/675, A61K 38/13, A61K 31/255, A61K 31/519, A61K 31/661, A61K 31/7076, A61P 41/00

(54) **TREATMENT OF HEMATOPOIETIC STEM CELL TRANSPLANT PATIENTS**
BEHANDLUNG VON PATIENTEN MIT EINER HÄMATOPOETISCHEN STAMMZELLTRANSPLANTATION
TRAITEMENT DE PATIENTS AYANT SUBI UNE GREFFE DE CELLULES SOUCHES HÉMATOPOÏÉTIQUES

(30) Priority: 08.03.2016 US 201662305003 P
(43) Date of publication of application: 16.01.2019
(73) Proprietor: Priothera Limited, Dublin 2 (IE)
(72) Inventor: BUCHER, Christoph, 4002 Basel (CH); GERGELY, Peter, 4002 Basel (CH); KATOPODIS, Andreas, 4002 Basel (CH); SMITH, Philip, 4002 Basel (CH)
(74) Representative: Plasseraud IP
(86) International application number: PCT/IB2017/051291
(87) International publication number: WO 2017/153889

(56) References cited:
- WO-A1-2014/128611
- SHIMIZU H ET AL: "KRP-203, a novel synthetic immunosuppressant, prolongs graft survival and attenuates chronic rejection in rat skin and heart allografts", CIRCULATION, AMERICAN HEART ASSOCIATION, INC, vol. 111, no. 2, 10 January 2005 (2005-01-10), pages 222-229, XP002596008, ISSN: 0009-7322, DOI: 10.1161/01.CIR.0000152101.41037.AB [retrieved on 2005-01-10]

## Description

The present invention relates to a compound for use in a method of treating patients who undergo hematopoietic stem cell transplantation (HSCT) with peripheral blood mobilized stem cells or blood marrow transplantation for hematological malignancies and for whom a faster engraftment is beneficial.

### BACKGROUND

Hematopoietic stem cell transplantation (HSCT) is the transplantation of multipotent hematopoietic stem cells, usually derived from bone marrow, peripheral blood, or umbilical cord blood. It may be autologous (the patient's own stem cells are used) or allogeneic (the stem cells come from a donor). It is a medical procedure in the field of hematology, and a potentially curative approach for a variety of malignant and nonmalignant hematopoietic diseases, such as e.g. cancers of the blood or bone marrow, e.g. lymphoma or leukemia. The cells that are transplanted can be unmodified or genetically engineered (e.g. Lentiviral, CRISPR). When HSCT is performed in patients with malignant disorders, preparative or conditioning regimens are administered before the transplantation in order to destroy or restrict the recipient's immune system (e.g. non-myeloablative, partial or full myeloablation) to prevent graft rejection and reduce the tumor burden. Such conditioning treatments may consist of performing radiation and/or chemotherapy.

Delivering (autologous or allogeneic) stem cells with a minimal toxicity is an unmet medical need because a substantial fraction of post transplant mortality and morbidity is related to conditioning toxicity. However, delivering stem cells after non-myeloablative conditioning has a risk of graft failure which prevents the successful outcome of a transplant procedure altogether.

In nonmalignant diseases the anti-neoplastic effect of conditioning is not needed.

Therefore there is a need to use a less myeloablative or less immunosuppressive conditioning treatment and/or limit the possible consequences thereof, e.g. side-effects, that are possibly associated with such conditioning regimens. Ideally, radiation and chemotherapy could be replaced by novel, non-toxic approaches entirely.

In some circumstances it would be advantageous to use a more reduced dose of hematopeitic cells for the transplantation than usually necessary for obtaining engraftment. But the use of less HSC would require a more intense conditioning, which has higher toxicity and provides a slower reconstitution.

Once hematopoietic stem cells have been transplanted the speed of engraftement plays an important and direct role for patients who have undergone blood marrow transplantation myeloablative conditioning, because every additional day of cytopenia (i.e. reduction in the number of blood cells), especially neutropenia (lower level of neutrophils) and thrombopenia (lower level of platelets), is usually associated with additional morbidity and mortality. This morbidity and mortality are caused by infections, bleeding and need for transfusion support both of red blood cells and platelets. Improving the engraftment, e.g. accelerating it, may also permit the patient to leave the hospital earlier and/or recover quicker fom the surgery.

Therefore there is a high unmet medical need to have a pharmaceutically effective drug which will make HSCT safer for both donor and recipient and may allow enlarging the patient populations that could benefit from HSCT. There is also a need to perform HSCT in patients who do not tolerate classical conditioning, e.g because the conditioning treatment would have severe or even life-threathening consequences on such patients, e.g. patients with metabolic diseases, ederly, juvenile patients or patients with comorbid conditions. It can also be patients for which it would not be justified to take the risks possibly associated with chemotherapy or radiotherapy of classical conditioning regimens in view of the nature of the disease affecting these patients, e.g. patients with non-malignant indications.

WO 2014/128611 A1 discloses that compound KRP203 can treat or prevent graft versus host disease (GVHD) in a patient who received hematopoietic stem cells (HSC) transplantation (HSCT) from a donor.

Unexpectedly, it was found that a compound according to formula (I), or a pharmaceutically acceptable salt thereof, and in particular KRP203, significantly increases the speed of HSC engraftment. Therefore the use of said compound permits to minimize the days of cytopenia to the shortest possible period and/or to use a lower cell dose or reduced conditioning. It also permits to render the HSCT easier and safer for the donor, and thus to identify donors more easily, to perform more transplantation and a larger panel of patients in need thereof.

### Summary of the Invention

In a first embodiment the present invention relates to a compound of formula (I) 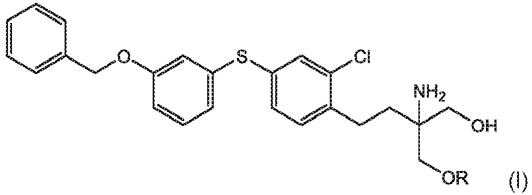 wherein R is H or P(O)₃H₂, or a pharmaceutically acceptable salt thereof for use of accelerating engraftement of hematopoietic stem cells in a patient who received a hematopoietic stem cell transplantation (HSCT) from a donor.

As used herein a compound of formula (I) or a pharmaceutically acceptable salt thereof, especially a hydrochloride salt, is selected from 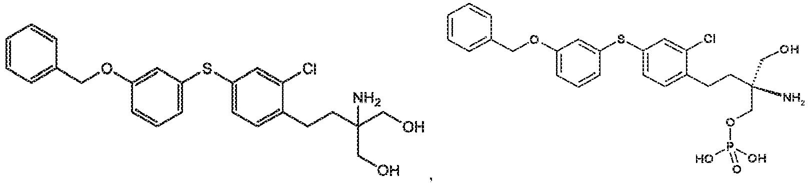 and

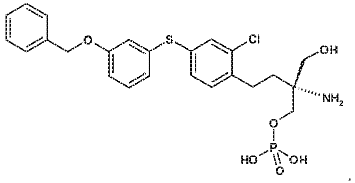

As used herein KRP203 refers to 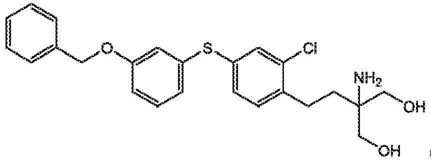 or a pharmaceutically acceptable salt thereof.

The term "pharmaceutically acceptable salts" refers to salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto.

As used herein a daily dosage refers to the daily amount of the pure active ingredient,i.e. a compound of formula (I) or a pharmaceutically acceptable salt thereof, e.g. 2-amino-2-[4-(3-benzyloxyphenylthio)-2-chloro-phenyl]ethyl-propane-1,3-diol hydrochloride.

As used herein the term "conditioning" or "conditioned" in the context of a patient pretreatment in need of HSCT typically means destroying substantially the bone marrow and immune system by a suitable procedure such as e.g. chemotherapy and/or radiation therapy.

Conditioning regimens have been classified as high-dose (myeloablative), reduced-intensity, and nonmyeloablative, following the Reduced-Intensity Conditioning Regimen Workshop, convened by the Center for International Blood and Marrow Transplant Research (CIBMTR) during the Bone Marrow Transplantation Tandem Meeting in 2006.

Accelerating engraftment according to the present invention, also permits to use a less myeloablative or lesss immunosuppressive conditioning treatment and/or limit the possible consequences, e.g. side effects, that are possibly associated with such conditioning regimens. In some circumstances, accelerating engraftment according to the present invention may even permit to perform HSCT without any conditioning.

Accelerating engraftment according to the present invention may also permit to use a reduced dose of hematopeitic cells for the transplantation than would otherwise be necessary for obtaining engraftment.

According to the invention, accelerating engraftment of hematopoietic stem cells transplantation may permit to use less HSC than otherwise require, e.g. without requiring a more intense conditioning.

Thus the present invention will make HSCT safer for both donor and recipient and may allow enlarging the patient populations that could benefit from HSCT. The present invention permits to perform HSCT in patients who do not tolerate a classical conditioning, e.g because the conditioning treatment would have severe or even life-threathening consequences on them, e.g. patients with metabolic diseases, ederly, juvenile patients or patients with comorbid conditions. The present invention also permits to perform HSCT in patients where taking the risks that can be associated with the chemotherapy or radiotherapy of the classical conditioning regimens would not be justified in view of the nature of their disease, e.g. patients with non-malignant indications. Because of the present invention, HSCT treatment can be utilized in a larger patients population, in particular in patients who could not have had access to such a technology because of their overall physical condition, age and/or the specific disease they are affected by.

As illustrative the following techniques can be used:
Non-myeloablative conditioning (NMA e.g. Mini-Seattle Conditioning), e.g. fludarabin or another chemotherapeutic agent typically at 30 mg/m2/day for three days followed by total body irradiation (TBI) typically at 1x 200cGy/day for one day;
Reduced intensity conditioning (RIC; typically FluBu);
Myeloablative conditioning, g (MAC; typically CyTBI of BuCy);
   or
e.g. high dose chemotherapy and total body irradiation (TBI) is typically performed according to national guidelines adapted to institutional practices, and includes the administration of fludarabin, busulphan.

The following dosing regimens are given as examples:
1) Fludarabin at 25 mg/m2/day i.v. x 3 days (for approximately 2-3 days) for a total dose of 75 mg/m2,
2) Busulphan at 0.8 mg/kg/6 h (for approximately 2 to 4 days),
3) Cyclophosphamide at 60 mg/kg/day i.v. x 2 days (approximately for 2 days) for a total dose of 120 mg/kg. To reduce the risk of CYC-induced hemorrhagic cystitis, patients will also receive high volume fluid flushes and mesna.
4) TBI will occur from approximately days 8 to 10 (days -8 and -1 relative to HSCT). Therecommended TBI dose is 200 cGy given twice daily for three days for a total dose of 1200 cGy.

### Detailed Description of the invention:

In a first embodiment, the present invention relates to of formula (I) as defined above, or a pharmaceutically acceptable salt thereof, e.g. KRP203 or a pharmaceutically acceptable salt thereof, e.g. KRP203 hydrochloride salt, for use in accelerating engraftment of hematopoietic stem cells in a patient who received said hematopoietic stem cells via a transplantation procedure from a donor.

As used herein, accelerating engraftment refers to obtaining, e.g. detecting, engraftment after the cell infusions, e.g. detecting the first day of engraftment, in a shorter period of time than without administering an effective amount of a compound of formula (I) as herein defined.

The first day of engraftment is usually defined as the 1^{st} day after a period of 3 consecutive days where the amount of neutrophils present in the blood, as determined e.g. by routine hematograph, is ≥ 500/µl.

Duration of engraftment also depends on the conditioning treatment that occurred or not before the transplantation. Without KRP203 therapy that period is usually around 15 to 16 days after cell infusion in case of full conditioning (myeloablative).

In a second embodiment, the present invention relates to a compound of formula (I) for the use according to the preceding embodiment, e.g. KRP203 or a pharmaceutically acceptable salt thereof, wherein said patient is first subjected to conditioning, e.g. myeloablative, reduced-intensity or nonmyeloablative, e.g. myeloablative conditioning, e.g destroying substantially all the bone marrow of the patient.

In a third embodiment, the present invention relates to a compound for the use according to any of the preceding embodiments, wherein said conditioning is a high dose chemotherapy comprising one or more agents selected from fludarabin, busulphan, methotrexate, cyclosporin A and cyclophosphamide.

In a fourth embodiment, the present invention relates to a compound for the use according to any of the preceding embodiments, wherein said conditioning is a total body irradiation (TBI) according to national guidelines.

In a fifth embodiment, the present invention relates to a compound for the use according to any of the preceding embodiments, wherein hematopoietic stem cell transplantation (HSCT) is carried out following to conditioning, e.g. immediately after conditioning, or 0 - 1 day after conditioning, or 1 - 8 days, or 1 - 10 days after conditioning.

In a sixth embodiment, the present invention relates to a compound for the use according to any of the preceding embodiments, wherein treatment of the patient with a compound of formula (I) is commenced 5 days before conditioning, in particular 3 days before conditioning, e.g. 1 day before conditioning.

In a further disclosure, the patient is subject to non-myeloablative conditioning or is not subjected to a conditioning regimen prior to the cell infusion, e.g. said patient is affected by a non-malignant condition, a non-hematological condition, is a juvenile, is an elderly patient, e.g. over 55 years old, or does suffer from a comorbid condition.

In a further disclosure, the patient does not undergo a conditioning regimen before the transplantation or does undergo a non-myeloablative conditioning, and optionally the patient is affected by a non-hematological condition.

In a further disclosure, the patient receives the cells from a donor, according to a method which comprises administering to said patient a number of donor cells up to 50% lower than otherwise needed without administering the compound of formula (I), e.g. up to 40% lower than otherwise needed without administering the compound of formula (I), e.g. up to 30% lower than otherwise needed without administering the compound of formula (I), e.g. up to 20% lower than otherwise needed without administering the compound of formula (I), e.g. up to 10% lower than otherwise needed without administering the compound of formula (I). Optionally the patient is subjected to a conditioning regimen before the transplantation, e.g. a non-myeloablative regmen.

In a further disclosure, the patient receives the cells from a donor, according to a method which comprises administering to said patient a number of CD34 cells comprised between about 1×10E6 cells/kg recipient to about 9×10E6 cells/kg recipient, e.g. about 1×10E6 cells/kg recipient to about 8×10E6 cells/kg recipient, e.g. about 1×10E6 cells/kg recipient to about 7×10E6 cells/kg recipient, e.g. about 1×10E6 cells/kg recipient to about 6×10E6 cells/kg recipient, e.g. about 1×10E6 cells/kg recipient to about 5×10E6 cells/kg recipient, e.g. about 1×10E6 cells/kg recipient to about 4×10E6 cells/kg recipient. Optionally the patient is subjected to a conditioning regimen before the transplantation, e.g. a non-myeloablative regmen.

In a further disclosure, the patient receives a dose of cells from the donor of about 1×10E6 cells/kg recipient, or about 2×10E6 cells/kg recipient, or about 2×10E6 cells/kg recipient, or about 2×10E6 cells/kg recipient or about 5×10E6 cells/kg recipient. Optionally the patient is subjected to a conditioning regimen before the transplantation, e.g. a non-myeloablative regmen. Optionally the patient is subjected to a conditioning regimen before the transplantation, e.g. a non-myeloablative regmen.

In a further disclosure, the period until the first day of engraftment is obtained within at least one day up to one week, e.g. at least one day, at least 2 days, at least 3 days, at least 4 days, at least 6 days.

In a further disclosure, the engraftment of hematopoeitic stem cells in a patient in need thereof is obtained in about 12 to 14 days, e.g. about 12 to 13 days, after the stem cell infusion, in particular wherein a conditioning treatment was performed before the cell infusion, e.g. a myeloblative or non myeloblative conditioning.

In a further disclosure, said patient is selected from a patient suffering from a malignant disease, a non-malignant indication, a metabolic disease, and a comorbid condition and an autoimmune disease, e.g. as herein defined, e.g. aplastic anemia, hemoglobulinopathy, thalassemia, Sickle disease, Hurler's disease.

In a further disclosure, the patient is selected from a patient suffering from a metabolic disease, e.g. hemoglobinopathy, aplastic anemia, thalassemia, Sickle disease, Hurler's disease, and/or an elderly patient, e.g. over 55 years old, or a juvenile patient, and said patient receives a compound of formula (I) or a pharmaceutically acceptable salt thereof, e.g. KRP203 or a pharmaceutically acceptable salt thereof, e.g. KRP203 hydrochloride. The patient may or may not receive a conditioning treatment before cell transplantation, e.g does receive a non-myeloablative conditioning regimen.

A further disclosure, which is not part of the present invention, describes a method for treating a metabolic disease, e.g. hemoglobinopathy, aplastic anemia, thalassemia, Sickle disease, Hurler's disease, in a patient undergoing hematopoietic stem cell transplantation (HSCT), which method comprises:
(i) Administering to the patient an effective amount of a compound of formula (I) or a pharmaceutically acceptable salt thereof, e.g. KRP203 or a pharmaceutically acceptable salt thereof, e.g. KRP203 hydrochloride;
(ii) Conditioning said patient prior the transplantation, e.g. performing a non-myeloablative conditioning; and
(iii) Transplanting hematopoietic stem cells from a donor to said patient.

A further disclosure, which is not part of the present invention, describes a method for performing hematopoietic stem cell transplantation (HSCT) in a patient in need thereof, e.g. a juvenile, an elderly (e.g. over 55 years old), which method comprises:
(i) Administering to the patient an effective amount of a compound of formula (I) or a pharmaceutical acceptable salt thereof, e.g. KRP203 or a pharmaceutical acceptable salt thereof, e.g. KRP203 hydrochloride;
(ii) Conditioning said patient prior the transplantation, e.g. performing a non-myeloablative conditioning; and
(iii) Transplanting hematopoietic stern cells from a donor to said patient.

In a further embodiment, the compound, e.g KRP203 or a pharmaceutically acceptable salt thereof, e.g KRP203 hydrochloride, is administered at a daily dose of 1, 2, 3, 4 or 5 mg (per patient), e.g. daily dose of 1 mg or 2 mg or 3 mg. The daily dose of the compound of formula (I), e.g. KRP203 or a pharmaceutically acceptable salt thereof, e.g. hydrochloride salt, may be administered once a day, or several times a day, e.g. once a day. The compound of formula (I), e.g. KRP203 or a pharmaceutically acceptable salt thereof, e.g. hydrochloride salt, may also be administered at longer intervals, e.g. once a week, or every 4-5 days. According to the invention, the donor may be the patient who receives his or her own stem cells (autologous transplantation), or another person (allogeneic transplantation). The source of the stem cell can be ombilical cord, haploidentical.

A further disclosure, which is not part of the present invention, describes a method or a compound in accordance to any of the preceding embodiments, wherein said compound is administered at a daily dose of 1, 2 or 3 mg (per patient). The daily dose of the compound of formula (I), e.g. KRP203 or a pharmaceutically acceptable salt thereof, may be administered once a day, or several times a day, e.g. once a day.

A further disclosure, which is not part of the present invention, describes a compound of formula (I), e.g. KRP203 or a pharmaceutically acceptable salt thereof, for use in performing HSCT in a patient in need thereof, comprising administering a daily dose of about 1mg to 3mg, e.g about 1mg, e.g. about 2 mg, e.g. about 3 mg.

A further disclosure, which is not part of the present invention, describes a method for performing HSCT in a patient in need thereof, comprising administering to a patient in need thereof compound of formula (I), e.g. KRP203 or a pharmaceutically acceptable salt thereof at a daily dose of about 1mg to 3mg, e.g about 1mg, e.g. about 2 mg, e.g. about 3 mg.

A further disclosure, which is not part of the present invention, describes a compound of formula (I), e.g. KRP203 or a pharmaceutically acceptable salt thereof, for use in treating a non-malignant condition or a non-hematological condition comprising administering a daily dose of about 1mg to 3mg, e.g about 1mg, e.g. about 2 mg, e.g. about 3 mg, and comprising performing HSCT in the patient. Optionally, the patient is a selected from a juvenile, an elderly patient and a patient who suffers from a comorbid condition, e.g. a patient over 55 years old.

A further disclosure, which is not part of the present invention, describes a method for treating a non-malignant condition or a non-hematological condition in a patient in need thereof, comprising the steps of i) performing HSCT in the patient and ii) administering to a patient in need thereof compound of formula (I), e.g. KRP203 or a pharmaceutical acceptable salt thereof at a daily dose of about 1mg to 3mg, e.g about 1mg, e.g. aboput 2 mg, e.g. about 3 mg. Optionally, the patient is a selected from a juvenile, an elderly patient and a patient who suffers from a comorbid condition, e.g. a patient over 55 years old.

The patient (receptor) may be affected by a metabolic disease, a malignant disease such as a blood cancer, e.g. by one disease selected from leukemia, multiple myeloma, lymphoma, e.g. acute lymphoblastic leukemia, acute myeloblastic leukemia, chronic lymphoid leukemia, myelodysplastic syndrome, non-Hodgkin lymphoma. In another embodiment, the patient is affected by a non-malignant disease, e.g. aplastic anemia; a metabolic disease or disorder, e.g. hemoglobinopathy, thalassemia, Sickle disease or Hurler's disease. The patient may have a comorbid condition, e.g. a heart disease, AIDS or cancer. The patient may be affected by bone marrow transplantation combined with solid organ transplantation for tolerance induction. The patient may suffer from autoimmune diseases such as e.g. type I diabetes, multiple sclerosis, scleroderma.

A further disclosure, which is not part of the present invention, describes a method for treating or preventing one disease amongst the diseases mentioned above, e.g. a malignant disease, a non-malignant disease, a comorbid condition or an autoimmune disease, wherein said method comprises:
(i) Administering to the patient an effective amount of a compound of formula (I) or a pharmaceutically acceptable salt thereof;
(ii) Transplanting hematopoietic stem cells from a donor to said patien; and optionally
(iii) Performing a conditioning to said patient prior the transplantation, e.g. performing a non-myeloablative conditioning. The compound of formula (I) or a pharmaceutical acceptable salt thereof, e.g. KRP203 or a pharmaceutically acceptable salt thereof, e.g. KRP203 hydrochloride, can be administered at a daily dose of about 0.5mg to 5mg, e.g. about 1 mg to about 3mg, e.g. about 0.5mg, e.g. about 1mg, e.g. about 3mg,

A further disclosure, which is not part of the present invention, describes a compound of formula (I) or a pharmaceutically acceptable salt thereof, e.g. KRP203 or a pharmaceutically acceptable salt thereof, e.g. KRP203 hydrochloride salt for use in a method of accelerating engraftment of hematopoietic stem cells in a patient who received hematopoietic stem cells via transplantation from a donor.

The patient may be medically infirm, elderly e.g. over 55 years old, e.g. over 60 years old), juvenile, e.g. children with severe combined immunodeficiency.

The compound of formula (I), e.g. KRP203 or a pharmaceutically acceptable salt thereof, e.g. KRP203 hydrochloride, may be administered after the transplantation, e.g. during up to 150 days post-transplantation, e.g. up to 120 days post-transplantation, e.g. up to 100 days post-transplant, e.g. during 3 months, e.g. during one months, e.g. during one week after transplantation. For example, the compound of formula (I), e.g. KRP203 or a pharmaceutically acceptable salt thereof, e.g. KRP203 hydrochloride, may be administered during about 120 days after the transplantation, e.g. about 110 days after the transplantation, e.g about 100 days after the transplantation, e.g about 90 days after the transplantation.

Administration of the compound of formula (I), e.g. KRP203 or a pharmaceutically acceptable salt thereof, e.g. KRP203 hydrochloride, can start before transplantation, e.g 1 to 3 days before the transplantation (e.g. one day, or two days before the transplantation), or at the day of transplantation. The duration of administering the compound of formula (I), e.g. KRP203 or a pharmaceutically acceptable salt thereof, e.g. KRP203 hydrochloride, can be of about 120 days, e.g. about 110 days, e.g about 100 days after the transplantation, e.g about 90 days.

The donor may the patient who receives his or her own stem cells (autologous transplantation), or another person (allogeneic transplantation).The patient (receptor) may be affected by one disease selected from leukemia, multiple myeloma, lymphoma, e.g. acute lymphoblastic leukemia, acute myeloblastic leukemia, chronic lymphoid leukemia, myelodysplastic syndrome, non-Hodgkin lymphoma.

### Treatment Procedure

Compound for accelerating engraftment of hematopoietic stem cells:
The compound of formula (I), in particular KRP203, especially compositions comprising 0,5; 1; 2; 3; 4 or 5mg of 2-amino-2-[4-(3-benzyloxyphenylthio)-2-chlorophenyl]ethyl-propane-1,3-diol or a pharmaceutically acceptable salt thereof are provided. Especially preferred are capsules or tablets comprising 1 or 2 mg 2-amino-2-[4-(3-benzyloxyphenylthio)-2-chloro-phenyi]ethyl-propane-1,3-diol, e.g. as hydrochloride.

The treatment may comprise (representative schedule):
A: A screening period (Days -50 to -2), Baseline (Day -1),
B: Drug treatment period from Day 1 to Day 111 and a follow-up period up to 365 days (from transplant), wherein the drug is a compound of formula (I) or a pharmaceutically acceptable salt thereof, e.g. 2-amino-2-[4-(3-benzyloxyphenylthio)-2-chloro-phenyl]ethyl-propane-1,3-diol, e.g. as hydrochloride,
C: Myeloablative or Mini Seattle Conditioning will be performed between Day 2 and Day 10 as per as described herein,
D: Transplanation (infusion of stem cells), i.e. HSCT will be performed on Day 11. Standard activities, in addition to the investigative treatment may include standard GVHD prophylaxis, pre and post transplant supportive care and follow-up assessments according to the institutional practices.

### Hematopoetic stem cells (HSC)

Peripheral mobilized stem cells will be used according to institutional practices. Suitable stem cell source must be available according to the graft selection algorithm as defined by JACIE* adapted to institutional standards using T-cell replete peripheral stem cells as a graft source. (*JACIE: The Joint Accreditation Committee Europe comprising the International Society for Cellular Therapy & European Group for Blood and Marrow Transplantation). In addition, the donor must be 9/10 or 10/10 matched with the recipient using molecular HLA matching techniques.

### Examples

### Example 1: mouse model of inherited metabolic disease (IMD)

Recipient C57BL/6 or IDUAKI (B6.129S-Iduatm1.1Kmke/J) mice were conditioned with a mild non-myeloablative regimen. A limited number of 1×10E3 HSC were transplanted from congenic mice. Groups were either untreated or treated with KRP for 15 days. More mice from the KRP203 treated group engrafted on day 56 and the engrafted mice had more donor cells than the untreated group. The conclusion is that in non-myeloablative conditions under limited HSC conditions, KRP treatment in the peri-transplant period augments engraftment.

### Example 2:

### Description of Experiment:

Day 1 = drug treatment commences (e.g. 2 mg KRP203 / per day)
Day 2 and Day 10: Conditioning was performed as described above (e.g. Mini Seattle or RIC)
Day 11: transplantation of stem cells from an allogeneic donor is being carried out
Day 11: pursuant to the foregoing transplantation procedure, the neutrophil count was determined and was 33% above baseline in the KRP203 group; and 11% above baseline for the control group
Day 15: neutrophil count was 100% above baseline for KRP203 group and 48% above baseline for control group.

To monitor for engraftment of donor hematopoietic stem cells in recipients, the neutrophil count is measured usually daily. The commonly accepted definition of "day of engraftment" (take) is the first of three consecutive days with a neutrophil count higher than 500 cells per microliter. A neutrophil count of higher than 500 cells per microliter is reached in the below experiment, when the probability of engraftement is 1.0 (100%) versus baseline or higher. The donor origin of these cells is normally confirmed by genomic analyses on day 30.

### Results

**Table 1**

| **Day** | **Probability of neutrophil engraftment Control** | **Probability of neutrophil engraftment KRP203** |
|---|---|---|
| 0 | 0.00 | 0.00 |
| 10 | 0.01 | 0.00 |
| 11 | 0.11 | 0.33 |
| 12 | 0.14 | 0.44 |
| 13 | 0.36 | 0.67 |
| 14 | 0.40 | 0.89 |
| 15 | 0.48 | 1.00 |
| 16 | 0.58 | 1.00 |
| 17 | 0.68 | 1.00 |
| 18 | 0.81 | 1.00 |
| 19 | 0.85 | 1.00 |
| 20 | 0.88 | 1.00 |
| 21 | 0.90 | 1.00 |
| 22 | 0.94 | 1.00 |
| 23 | 0.99 | 1.00 |

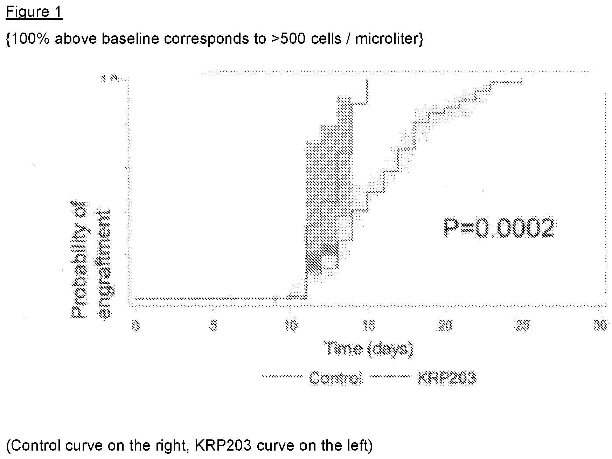

Figure 1 and Table 1 show the probability of neutrophil engraftment of the control group versus the group of patients treated with KRP203. As it can be seen, the group treated with KRP203 engrafted much faster than the control group.

## Claims

1. A compound of formula (I) 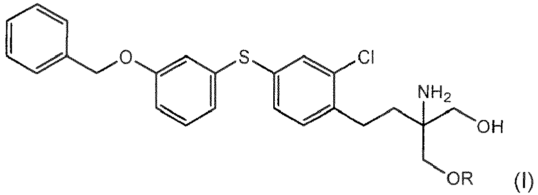
wherein R is H or P(O)₃H₂,
or a pharmaceutically acceptable salt thereof for use in accelerating engraftment of hematopoietic stem cells in a patient who received said hematopoietic stem cells via a transplantation procedure from a donor.

2. A compound for use according to claim 1, wherein said patient is first subjected to conditioning thereby destroying substantially all the bone marrow of the patient.

3. A compound for use in accordance to claim 2, wherein said conditioning is a high dose chemotherapy comprising one or more agents selected from fludarabin, busulphan, methotrexate, cyclosporin A and cyclophosphamide.

4. A compound for use in accordance to claim 2 or 3, wherein said conditioning is a total body irradiation (TBI) according to national guidelines.

5. A compound for use in accordance to any of claims 2 to 4, wherein hematopoietic stem cell transplantation (HSCT) is carried out following to conditioning, e.g. immediately after conditioning, or 0 - 1 day after conditioning, or 1 - 8 days, or 1 - 10 days after conditioning.

6. A compound for use in accordance to any of claim 2 to 5, wherein treatment of the patient with a compound of formula (I) is commenced 5 days before conditioning, in particular 3 days before conditioning and especially 1 day before conditioning.

7. A compound for use in accordance to any of the preceding claims, wherein said compound or a pharmaceutically acceptable salt thereof is selected from 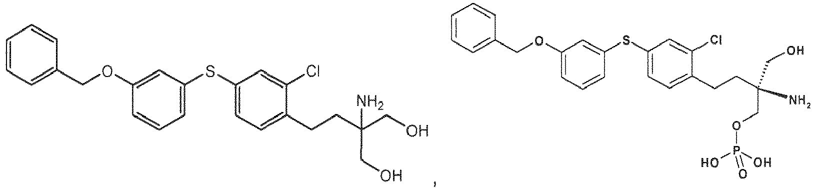 and

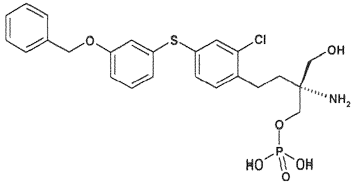

8. A compound for use in accordance to any of the preceding claims, wherein said compound or a pharmaceutically acceptable salt thereof is KRP203 or the hydrochloride salt thereof.

9. A compound for use in accordance to any of the preceding claims, wherein said compound is administered at a daily dose of 1, 2, 3 4 or 5mg (per patient).

## Patentansprüche

1. Verbindung der Formel (I) 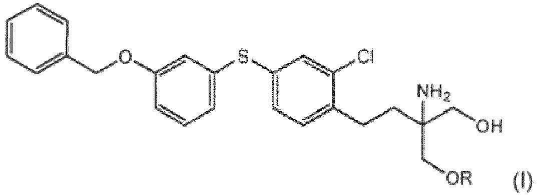
wobei R H oder P(O)₃H₂ ist,
oder ein pharmazeutisch verträgliches Salz davon zur Verwendung beim Beschleunigen des Engraftments von hämatopoetischen Stammzellen bei einem Patienten, der die hämatopoetischen Stammzellen über ein Transplantationsverfahren von einem Spender erhalten hat.

2. Verbindung zur Verwendung nach Anspruch 1, wobei der Patient zuerst einer Konditionierung unterzogen wird, wobei das gesamte Knochenmark des Patienten im Wesentlichen zerstört wird.

3. Verbindung zur Verwendung nach Anspruch 2, wobei die Konditionierung eine Hochdosis-Chemotherapie ist, die ein oder mehrere Agenzien umfasst, die aus Fludarabin, Busulphan, Methotrexat, Cyclosporin A und Cyclophosphamid ausgewählt sind.

4. Verbindung zur Verwendung nach Anspruch 2 oder 3, wobei die Konditionierung eine Ganzkörperbestrahlung (total body irradiation, TBI) nach den nationalen Richtlinien ist.

5. Verbindung zur Verwendung nach einem der Ansprüche 2 bis 4, wobei die Transplantation von hämatopoetischen Stammzellen (HSCT) nach der Konditionierung, z.B. sofort nach der Konditionierung oder 0-1 Tage nach der Konditionierung oder 1-8 Tage oder 1-10 nach der Konditionierung durchgeführt wird.

6. Verbindung zur Verwendung nach einem der Ansprüche 2 bis 5, wobei die Behandlung des Patienten mit der Verbindung der Formel (I) 5 Tage vor der Konditionierung begonnen wird, insbesondere 3 Tage vor der Konditionierung und insbesondere 1 Tag vor der Konditionierung.

7. Verbindung zur Verwendung nach einem der vorhergehenden Ansprüche, wobei die Verbindung oder ein pharmazeutisch verträgliches Salz davon ausgewählt ist aus 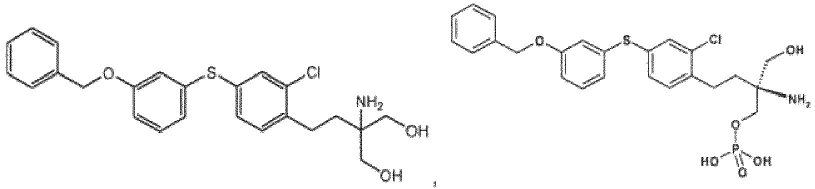 und

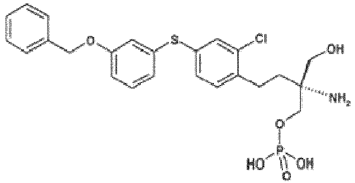

8. Verbindung zur Verwendung nach einem der vorhergehenden Ansprüche, wobei die Verbindung oder ein pharmazeutisch verträgliches Salz davon KRP203 oder das Hydrochloridsalz davon ist.

9. Verbindung zur Verwendung nach einem der vorhergehenden Ansprüche, wobei die Verbindung mit einer täglichen Dosis von 1, 2, 3, 4 oder 5 mg (pro Patient) verabreicht wird.

## Revendications

1. Composé de formule (I) 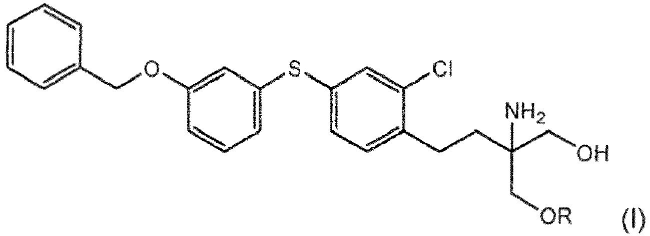
dans lequel R est H ou P(O)₃H₂,
ou un sel pharmaceutiquement acceptable de celui-ci pour son utilisation dans l'accélération de la prise de greffe de cellules souches hématopoïétiques chez un patient qui a reçu d'un donneur lesdites cellules souches hématopoïétiques par l'intermédiaire d'une procédure de transplantation.

2. Composé pour son utilisation selon la revendication 1, dans lequel ledit patient est d'abord soumis à un conditionnement détruisant ainsi sensiblement toute la moelle osseuse du patient.

3. Composé pour son utilisation selon la revendication 2, dans lequel ledit conditionnement est une chimiothérapie à dose élevée comprenant un ou plusieurs agents sélectionnés parmi la fludarabine, le busulfan, le méthotrexate, la cyclosporine A et le cyclophosphamide.

4. Composé pour son utilisation selon la revendication 2 ou 3, dans lequel ledit conditionnement est une irradiation corporelle totale (ICT) conformément aux lignes directrices nationales.

5. Composé pour son utilisation selon l'une quelconque des revendications 2 à 4, dans lequel la transplantation de cellules souches hématopoïétiques (HSCT) est réalisée après le conditionnement, par exemple immédiatement après le conditionnement, ou 0 à 1 jour après le conditionnement, ou 1 à 8 jours, ou 1 à 10 jours après le conditionnement.

6. Composé pour son utilisation selon l'une quelconque des revendications 2 à 5, dans lequel le traitement du patient avec un composé de formule (I) est débuté 5 jours avant le conditionnement, en particulier 3 jours avant le conditionnement et notamment 1 jour avant le conditionnement.

7. Composé pour son utilisation selon l'une quelconque des revendications précédentes, dans lequel ledit composé ou un sel pharmaceutiquement acceptable de celui-ci est sélectionné parmi 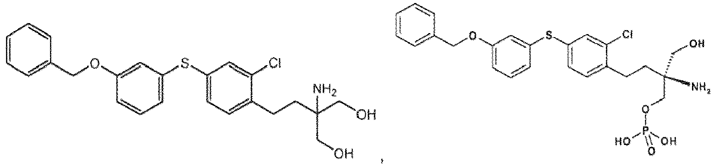 et

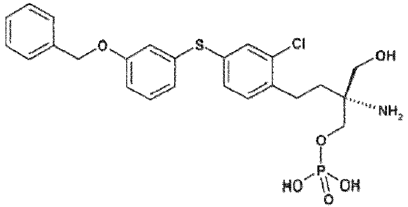

8. Composé pour son utilisation selon l'une quelconque des revendications précédentes, dans lequel ledit composé ou un sel pharmaceutiquement acceptable de celui-ci est KRP203 ou le sel de chlorhydrate de celui-ci.

9. Composé pour son utilisation selon l'une quelconque des revendications précédentes, dans lequel ledit composé est administré en une dose quotidienne de 1, 2, 3, 4 ou 5 mg (par patient).
